(19) Europäisches Patentamt
European Patent Office
Office européen des brevets

(11) **EP 3 900 631 A1**

(12) **EUROPEAN PATENT APPLICATION**
published in accordance with Art. 153(4) EPC

(43) Date of publication:
**27.10.2021 Bulletin 2021/43**

(51) Int Cl.:
***A61B 5/16*** *(2006.01)*     ***A61B 5/0408*** *(2006.01)*

(21) Application number: **18917043.4**

(22) Date of filing: **21.12.2018**

(86) International application number:
**PCT/RU2018/000848**

(87) International publication number:
**WO 2020/130870 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Aktsionernoye Obschestvo "neyrotrend"**
**Moscow 143026 (RU)**

(72) Inventors:
• **ADIATULLIN, Adel Vladimirovich**
 **Nizhnekamsky r-n**
 **Res. Tatarstan**
 **g. Nizhnekamsk**
 **423578 (RU)**

• **NAUMOVA, Anna Aleksandrovna**
 **Tulskaya obl.**
 **g. Tula**
 **300002 (RU)**
• **SEROV, Igor Evgenyevich**
 **Moskovskaya oblast**
 **Krasnogorsky r-n**
 **g. Krasnogorsk**
 **143405 (RU)**
• **KOLKOVA, Kseniya Mikhaylovna**
 **Moskva**
 **125373 (RU)**

(74) Representative: **Spengler, Robert**
 **Potthast & Spengler**
 **Patentanwälte PartG mbB**
 **Magirus-Deutz-Straße 12**
 **89077 Ulm (DE)**

(54) **METHOD FOR MEASURING THE MEMORABILITY OF A MULTIMEDIA MESSAGE**

(57) The present technology measures memorability of multimedia messages by users. A "10-20 electrode system" is installed on the head of a user and reference electrodes are installed on each earlobe or mastoid of the user. A multimedia message is presented to the user and, concurrently, a brain activity index of the user is recorded using the 10-20 electrode system and the reference electrodes. A memorability of the multimedia message by the user is evaluated based on the recorded brain activity. This evaluation may include a determination of a leads entropy measured using a first electrode of the 10-20 electrode system along with the reference electrodes, and a determination of coherence leads determined based on various pairs of electrodes of the 10-20 electrode system along with the reference electrodes.

Figure 1

EP 3 900 631 A1

## Description

### Cross-Reference

The present application is a National Phase Entry of International Application no PCT/RU2018/000848, filed on December 21, 2018, and claims priority to Russian Patent Application No. 2018145585, filed on December 21, 2018, the entirety of each of which is incorporated herein by reference in its entirety.

### Technical Field

The present technology belongs to the means of objective quantitative assessment of impact on the user from multimedia messages (particularly, promotional videos); it may be used for development of training aids, preparation of promotional material, and also for assessment of awareness and memorability for videoclips.

### Background Art

There is a known method (see CN103500184, published 13.09.2013) for the assessment of video content memorability relying on person's visual and audio learning styles, which may be used for assessment of videoclips memorability and may be used in advertising and news-making industries.

There also exists the method (see CN102855630, published 21.08.2012) for the assessment of images memorability based on noticeability entropy and characteristic features of the objects' set.

The major fault of both aforementioned methods is such that the measured index of memorability has no direct relation on the possibility of reproduction or recognition of the information retained by the user and concerned to the researchable object.

### Summary

The technical result of the present technology is the reduction of the labor effort required for assessment of the impact of multimedia messages on the users, particularly concerning the messages memorability; accordingly, there will be less time consumed to prepare or correct the materials where the objects that required quick memorability and/or reliable recognition and/or awareness are used. The second technical result is the ability to perform the objective universal quantitative assessment of the memorability indexes.

The present technology is based on the known fact that the recollection of the objects seen or heard takes some time, and test subjects often prefer to report that they did not remember the object rather than spend some time for its memorability. Nonetheless, the research has shown that the reaction of test subjects for the object that might be remembered and the object permanently forgotten, differs even at the moment of the contact of the first test subject with the object.

According to one of implementation variants, there is provided the measurement method of memorability for multimedia messages based on "10-20 system" use and reference electrodes installed at user's earlobes or mastoids; the electrodes must be installed at the points C3, C4, T3, T4, P3 and F4 (identified according to 10-20% international electrodes arrangement scheme) of the user's head; the user's brain activity index will be registered during multimedia message presentation to the user; based on registration results, there will be determined the values for: leads entropy (E1) for C3 electrode; leads' first coherence (K1), that is equal to leads' coherence for T3 and T4 electrodes within the range 30-45 Hz; leads' second coherence (K2), that is equal to leads' coherence for C4 and T4 electrodes within the range 4-8 Hz; leads' third coherence (K3) that is equal to leads' coherence for P3 and F4 electrodes within the range 8-13 Hz; there will be specified the memorability's weighting factor for the E1 (KE1); there will be specified first, second and third memorability's weighting factors, respectively, for K1, K2 and K3 (KR1, KR2 and KR3, respectively), and there will be defined the multimedia message's memorability, or "recall" (Rec) via calculation of the sum of productions $KE1*E1$, $KR1*K1$, $KR2*K2$ and $KR3*K3$ for E1, K1, K2 and K3, calculated for the multimedia message.

According to the particular implementation variant, there will be estimated the base memorability, or "base recall" (BRec) via calculation of the sum of productions $KE1*E1$, $KR1*K1$, $KR2*K2$ and $KR3*K3$ for E1, K1, K2 and K3, calculated for the reference multimedia message with predefined retention probability, accepting that multimedia message retention probability is higher than reference multimedia message retention probability at BRec higher than Rec, and lower than reference multimedia message retention probability at BRec lower than Rec.

For the one particular implementation variant, the reference multimedia message will be presented to several users, and the reference multimedia message retention probability will be calculated as amount of users who retained the message, divided by the total amount of users both retained and not retained the reference multimedia message.

For the one particular implementation variant, the multimedia message will be the promotional video, and the reference multimedia message will be the promotional video too.

For the one particular implementation variant, there will be chosen KR1 < KR3 < KR2 < KE1.

For the one particular implementation variant, there will be chosen KR1 = 0,5±30%, KR3 = 1±30%, KR2 = 2±30%, KE1 = 4±30%.

For the one particular implementation variant, the multimedia message retention probability will be 50% at Rec=2.102.

For the one particular implementation variant, the multimedia message retention probability (P) will be determined from such formula:

$$P = \frac{1}{\left(1 + e^{(2.102 - KE1*E1 - KR1*K1 - KR2*K2 - KR3*K3)}\right)} \cdot$$

For the one particular implementation variant, the multimedia message will be presented to the user in the sequence of messages that includes additional multimedia messages; herein the additional multimedia messages will be placed at the start and at the end of sequence.

For the one particular implementation variant, the sequence of messages includes pauses between additional multimedia messages, also between the additional multimedia messages and multimedia message.

For the one particular implementation variant, the pauses will be filled by neutral videoclips.

For the one particular implementation variant, during the assessment of memorability there will be no exclusion of artifacts from electroencephalograms due user's spontaneous behavior.

For the one particular implementation variant, there will be registered the user's eyelids' position via the video monitoring devices, and the results at the closed position of the user's eyelids lasting for more than 2 seconds will be excluded from the electroencephalograms.

For the one particular implementation variant, for the additional multimedia message, subsequent to the results of presentation to at least 10 users, there defined the average BRec and BRec defined for user, shall consider the unreliable if the divergence of average BRec and BRec defined for user is more than 20% with respect to average BRec.

## Brief Description of the Drawings

Additional objects, aspects and advantages of the present technology will come across after reading of the following description, with reference to the attached drawings, where:

**Fig. 1** shows a sample variant of the system implementing the present technology;

**Fig. 2** shows a location of the electrodes according to 10-20% international electrodes arrangement scheme ("10-20 system");

**Fig. 3** shows a sample flow-chart of implementation of the proposed method;

**Figs. 4a** and **4b** show a sample variant of electrode supports;

**Fig. 5** shows a variant of the present system implementation, including the user equipment;

**Fig. 6** shows an example of computer system of general purpose.

## Detailed Description

Objects and aspects of the present technology, the approaches used to reach these objects and aspects, will be clear by reference to sample implementation variants. However the present technology is not limited to the sample implementation variants disclosed below; it also may be implemented via different variants.

The terms "component", "element", "system", "module", "part" (including "integrated part"), "block" and similar used in this Description, are used for identification of computer entities (for example, objects related to the computer, computational entities), that may be the hardware (in particular the equipment - for example, the device, tool, instrument, facility, device's integral part, including processor, microchip, printed circuit board etc.), the software (for example, executable program code, compiled application, program module, software and/or code part etc.) or firmware. For instance, the component may be the process executed by the processor, the processor itself, the object, the executable file, the program, the function, the method, the library, sub-program and/or computing device (for example, microcomputer or PC) or hardware or software complex. As an illustration: just as application that has been run on server (in particular, on central server) may be the component or module, so the server may be the component or module, too. At least one component may be located inside the process. The component may be located at the one computing device (for example, PC) and/or may be shared between two and more computing devices. For instance, in a particular case the application (component) may be introduced as server component (server part) and client component (client part). In a particular case the client component may be installed at least in one computing device, and the server component may be installed at the second computing device, from which, in a particular case, there will be performed the control and/or setting-up of the first computing device (and/or its integrated components/parts). In a particular case the module (and etc.) may be implemented as one file or the set of files, including the executable file (or files), which in turn may be linked, at least, with one software library (for example, implemented as dll-file, which is the compiled form of Dynamic Link Library), and, at least, with one file (for example, containing the application's service data, application's meta-data, the data useful for application's functioning), and/or local and/or remote service (for example, web-service, including applications and services built on service-oriented architecture/SOA, including, but no limited to, REST - Representational State Transfer - technologies, RPC - Remote Procedure Call, and other).

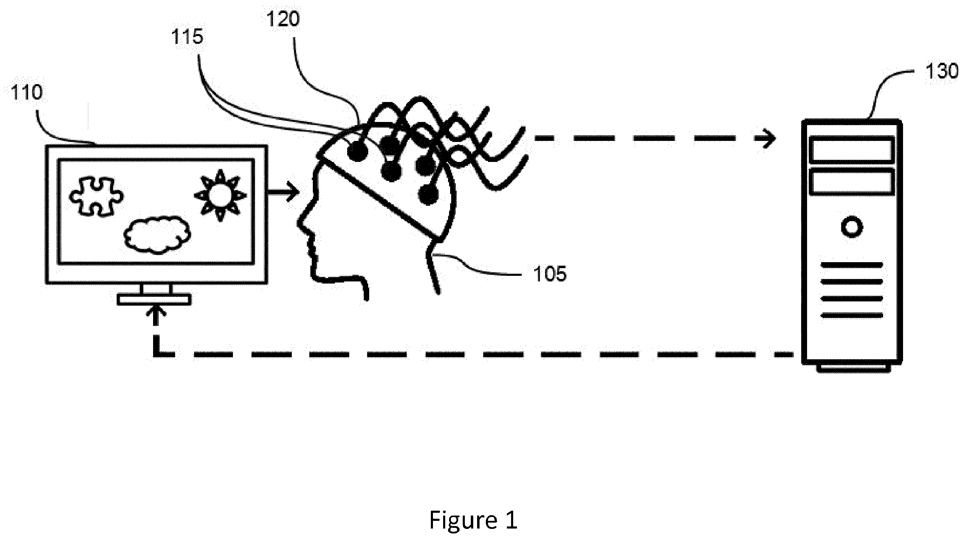

The **Fig. 1** depicts a sample variant of the system implementing the present technology.

The present system (shown on **Fig. 1**) includes a tool for electroencephalogram reading/registration **120** (linked with an electronic digital device - for example, a computer **130**), and a display **110** (linked with the computer **130**), with the aid of which the user **105** will be presented with at least one multimedia message. The multimedia message may include video images, music and text. The multimedia message comprises data saved at least in one file, for example - FullHD (1920x1080) video in AVI, XVid, MP4, WMV, MKV, etc. format. In a particular case the quality, sound level and duration of the multimedia messages are the same for all presented multimedia messages. In a particular case the display **110** supports the frame refresh with frequency at least 40 Hz and up to 150 Hz, and response time not exceeding 7 millisecond (ms). In a particular case the electronic digital device (for example computer **130** and display **110**) may be implemented as single device - for example, all-in-one PC, PC pad, smart phone etc.

The tool for electroencephalogram's reading - i.e. brain electrical activity registration tool - **120**, is used for registration of the results of electroencephalogram (EEG) of user **105**. Display **110** is intended for presentation of the objects - for example, multimedia messages. In a particular case of implementation the user **105** may be presented with several multimedia messages.

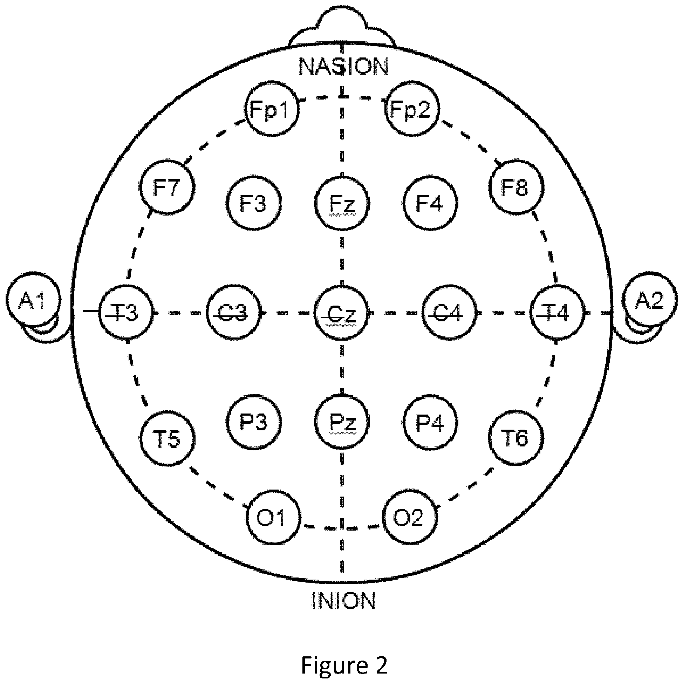

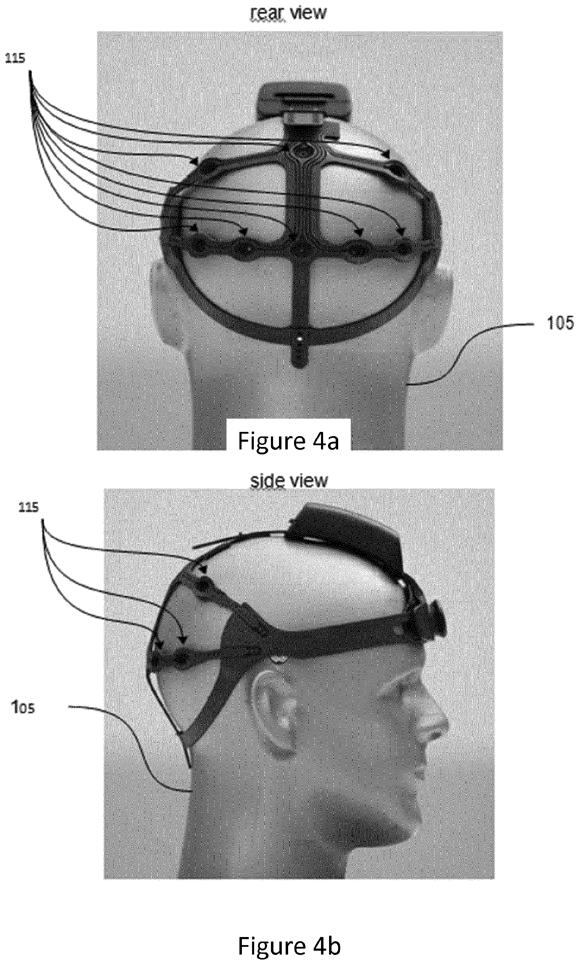

The system shown at **Fig. 1** includes and uses the electrodes **115** configured to provide contact with the user's scalp to transfer corresponding potentials to the analog data processing block. The electrodes may be fixed at the electrode supports on the user's head. In the present technology, the electrodes are arranged according to 10-20% international electrodes arrangement scheme ("10-20 system" or "10-20 scheme"), wherefore the description and the claims of the present technology use the terminology of the specified system; however the interpretation of the aspects of the present technology's claims may consider that in a particular case there may be no possibility to achieve an absolutely precise arrangement of the electrodes according to the "10-20 scheme", and the electrodes may be located at the areas having radiuses from 2 to 7 mm and centers located at the points estimated according to the "10-20 scheme". See **Fig. 2** for the detailed arrangement of the electrodes according to the "10-20 scheme". For implementation of the present technology, every single electrode may be separately glued, or the coupling may be implemented as sets of electrode supports - as in the case, for example, of elastic skull-caps with fixed electrodes, in the case of helmets or other solid or elastic frames allowing the possible arrangement of electrodes as part of implementing of the present technology. See **Fig. 4** for a sample variant of electrode supports. The electrodes may be located in close proximity and linked via conductors with an analog data processing block that transforms analog data to digital data according to a predefined algorithm ensuring analog to digital conversion with predefined time periods. Digital data from the analog data processing block may be transmitted to computer **130** via wired interface or via wireless communications.

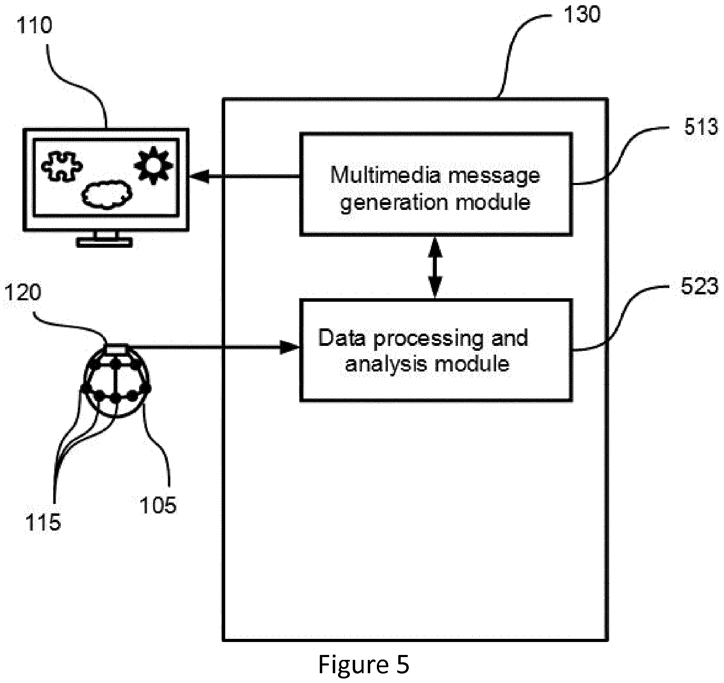

The electronic digital device implemented for example as portable or desktop computer **130** provides the analysis of the brain activity subsequent to the measurement results. Activity analysis is performed, for example, by making use of the data processing and analysis module **(523, Fig. 5)**. For analysis the electroencephalograms (EEG) and data concerning the parameters of the at least one generated multimedia message are used. Multimedia message generation is performed via multimedia message generation module **(513, Fig. 5)** that generates data useful to present multimedia message on display, and specifically at specified moments, providing the presentation of multimedia messages on the display (screen) **110**. The moments of generation (time of creation), moments of blanking (time of disappearance) and other characteristics or parameters of generated multimedia messages may be defined automatically by multimedia message generation module **(513, Fig. 5)**. Multimedia messages parameters may be specified as result of the machine learning, including, optionally, the use of artificial intelligence. The time of creation, also the time of disappearance or blanking, and other characteristics of generated multimedia messages may be specified via multimedia message generation module **(513, Fig. 5)**.

The tool for electroencephalogram's reading **120** - i.e. brain electrical activity registration tool (EEG) - is provided with attachment for electrical potential sensors on the user's head and allows EEG analyzing. It is recommended to use tools that allow registration of the electrical potential with frequency no less than 125 cycles per second, allowing (via data processing and analysis module **(523, Fig. 5)** use and/or via data processing module that may be the part of the tool for electroencephalogram's reading, encephalograph **120**) to detect the specific components of EEG alterations at the user's attention focusing on objects, in particular - the multimedia messages presented at display.

Using the integrated tool for electroencephalogram's reading **120** and/or computer **130** elements of processing and analysis that, for example, realizes the artificial intelligence structures, there occurs the possibility of processing and interpreting of the registered data, as described in the present description. Aforementioned elements of processing and analysis, including the artificial intelligence tools, may be part of the data processing and analysis module **(523, Fig. 5)** or part of the computer **130** module linked with the tool for electroencephalogram reading **120** or other module - for example, with computer **130** module, including the data processing and analysis module **(523, Fig. 5).** The data processing and analysis module **(523, Fig. 5)** or at least one of its parts may be the part of the tool for electroencephalogram reading **120**.

The system shown on **Fig. 1** uses an algorithm that is using the user's brain characteristic features to change the electrical parameters of the brain activity, registered at presentation of multimedia message to the user if the user's attention is focused on the message, wherein the changes of electrical parameters of user's brain activity may be registered via the tool for electroencephalogram's reading **120**.

The system shown in **Fig. 1** provides presentation of multimedia messages at the display **110;** every message characteristically exhibits a unique feature set having an unique effect on the electrical parameters of user's **105** brain activity.

The received brain activity data (in particular, data that are registering or registered) may be processed (in full or in part) either by computer **130** (in particular, at least by one of its module - for example, by data processing and analysis module **(523, Fig. 5)** and/or by the tool for electroencephalogram's reading **120** (or at least by one of its modules when such tool (module, device etc.) for electroencephalogram's reading **120** allows to perform such processing - for example using, at least, one processor, microprocessor, or the controller, microcontroller etc.)

On **Fig. 2** there is shown the location of the electrodes according to 10-20% international electrodes arrangement scheme ("10-20 system" or "10-20 scheme").

Positioning of the electrodes the "10-20 scheme" uses the measurement of the distances from the skull bone markers with subsequent percentage calculation of the intervals between electrodes. In this regard:

1) The distance between Nasion and Inion points is measured. The Oz point and Occipital electrodes line (O1, O2) are positioned at 10% from this measured distance above the Inion. In front of this line, the Pz point and Parietal electrodes line (P3, P4) are positioned at a distance of 20%. The Cz point and central electrodes line (C3, C4) are positioned at a further 20% - the Fz point and frontal electrode line (F3, F4) are positioned at a still further 20%. Pre-frontal electrodes (Fp1 and Fp2) are positioned on a line that is located 10% above the Nasion point and at 20% distance from the frontal electrode line. The Fpz point is positioned at the cross-over of this line with a longitudinal line extending between the Nasion and Inion points;

2) the second base distance is measured between parotid points (the landmark is the recess just above the antilobium) via the line that is running across the center of the first distance. The distance also divides into sections in percents: at 10% above auditory passways from both sides there located the temporal electrodes (T3 and T4), the aforementioned central electrodes (C3, C4) are located at 20% above the temporal electrodes ;

3) the third distance is measured as head circumference, the tape being laid carefully through already marked points Fpz, T3, Oz and T4 (circumferentially). The value 100% is set as half of the distance measured and from there the 10% to the left and to the right of Fpz are calculated to position the pre-frontal electrodes (Fp1 and Fp2, respectively), and 10% - from Oz, to position the occipital electrodes (O1 and O2). On this line there also positioned:

- the frontal electrodes (F7 and F8), at the interval 20% from Fp1 (behind) and T3 (to the fore), other side - similar.

- the temporal electrodes (T5 and T6), at the interval 20% from T3 (behind) and O1 (to the fore), other side - similar.

At the midline there positioned the sagittal electrodes: frontal (Fz), central (Cz), parietal (Pz).

As reference electrodes, electrodes A1 and A2 positioned on test subjects' earlobes or mastoids are used; herewith the term "leads" will be understood as registration of the difference of potentials between an electrode and a corresponding reference electrode A1 or A2 located at the earlobe on the same side of the user's head.

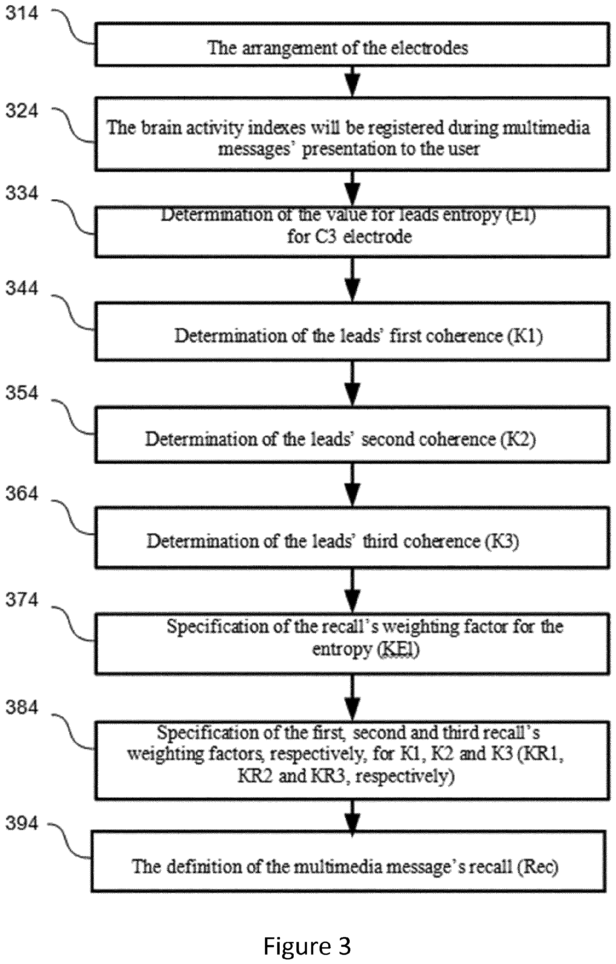

On **Fig. 3,** there is depicted a sample flow-chart of implementation of the method i accordance with the non-limiting embodiments of the present technology.

The described method allows the reliable ranging of the measurement results.

At the Step **314** (using the "10-20 system" and with reference electrodes positioned at user's **105** earlobes or mastoids) the electrodes located at the C3, C4, T3, T4, P3 and F4 points are arranged on the user's head, defined according to 10-20% international electrodes arrangement scheme C4-T4.

In a particular case before the presentation of the multimedia message to the user, the user fills a questionnaire where he enters: full name, gender, dominant arm, dominant eye, age, education, user's economic activity (when the user has a job), marital status, the number of children, financial status, the questions concerned the presented multimedia messages (for example, the rate of consumption of the goods/products that multimedia message promotes, etc.).

The user is also invited to specify some user's individual body built peculiarities or characteristics of current health that may affect the results of multimedia messages' presentation or processing of the registered data. Such peculiarities or characteristics may be: cerebropathy condition, head injuries, convulsive attacks condition, epistaxis, recent sleep disturbances or long-lasting sleeplessness, health degradation on the day before or at the day of multimedia messages' presentation, increased nervous and emotional tension on the day before or on the day of multimedia messages' presentation, alcohol or drugs ingestion on the day before or on the day of multimedia messages' presentation, strong tea or coffee ingestion on the day before or on the day of multimedia messages' presentation.

At the Step **324** (using the display **110**) the user **105** is presented with at least one multimedia message; the user's **105** brain activity indexes are registered using the electrodes as arrange at Step **314,** the electrodes being linked with the tool for electroencephalogram's reading **120** and/or computer **130**. In one particular case the multimedia message is presented to the user **105** in the sequence of (multimedia) messages that includes additional multimedia messages; herein the additional multimedia messages may be placed at the start and at the end of sequence. In one particular case, at least one user may be presented with at least one multimedia message and at least two additional multimedia messages. In one particular case, if there may be no special task of comparison of multimedia messages for different goods categories, the multimedia messages, including the additional multimedia messages, belonging to the good's category. In one particular case, if there may be no special task of comparison of multimedia messages for different price categories of goods, the multimedia messages, including the additional multimedia messages, belonging to the good's price category. In one particular case, if there may be no special task of comparison of multimedia messages of different artistic presentation, the multimedia messages, including the additional multimedia messages, being of the same category of artistic presentation.

In one particular case, the aforementioned sequence of messages includes pauses between additional multimedia messages, also between the additional multimedia messages and multimedia message. In one particular case, the pauses may be filled by neutral videoclips.

In one particular case, the aforementioned sequence of messages includes at least one neutral ambient multimedia message, for example, an image of the clouded sky. The neutral ambient multimedia message used for reading (registration) of the user's neurophysiologic parameters in quiescent state may be used for smoothing of the impact of the previous multimedia message.

In one particular case, the aforementioned sequence of messages includes a start (introductory) multimedia message with instructions for user - for example, "close your eyes", "open your eyes", "look at the screen" etc.

At the Step **334** the value for leads entropy (E1) for C3 electrode is determined.

In one particular case the leads entropy may be the measure of relative entropy (the entropy value in fraction of the possible maximum) and may be calculated by the following formula:

$$H\% = \frac{H(x)}{LogN} * 100\%$$

where: N is the number of the possible outcomes of the random value X; and

H(x) is the measure of the informational entropy;

The measure of the informational entropy, when the random value varies from $-\infty$ to $+\infty$, having the maximum entropy at given dispersion $\sigma^2$, is the normal (Gaussian) distribution with entropy calculated using the following formula:

$$H(x) = Log_2 \sqrt{2\pi e \sigma^2}$$

The procedure of probability-distribution entropy calculation for one lead includes:

1) the maximum and minimum of the EEG sections are read without artifacts;

2) given that the level noise from pick point to pick point is usually 1.5-2 Microvolt (mkV), for the table (histogram) of the EEG amplitude distribution (from isoline) an interval of 2 mkV is set. Therefore, the EEG amplitude is deemed as 0 if it falls within the range from -1 up to 1 mkV;

3) The P(Xi) - probability of the outcome i - is a relative frequency for corresponding column.

At the Step **344** the leads' first coherence (K1) that is equal to leads' coherence for T3 and T4 electrodes within the range 30-45 Hz is determined.

At the Step **354** the leads' second coherence (K2) that is equal to leads' coherence for C4 and T4 electrodes within the range 4-8 Hz is determined.

At the Step **364** the leads' third coherence (K3), that is equal to leads' coherence for P3 and F4 electrodes within the range 8-13 Hz is determined.

At the Step **374** the memorability's weighting factor for the E1 (KE1) is specified.

At the Step **384** first, second and third memorability's weighting factors, respectively, for K1, K2 and K3 (KR1, KR2 and KR3, respectively) are specified. In one particular case KR1, KR3, KR2 and KE1 will be chosen such that KR1 < KR3 < KR2 < KE1. In one particular case, KR1 = $0.5\pm30\%$, KR3 = $1\pm30\%$, KR2 = $2\pm30\%$, KE1 = $4\pm30\%$.

At the Step **394** the multimedia message's memorability (Rec) is determined via calculation of the sum of productions KE1$*$E1, KR1$*$K1, KR2$*$K2 and KR3$*$K3 for E1, K1, K2 and K3, specified for the multimedia message.

In one particular case, the base memorability (BRec) may be estimated via calculation of the sum of productions KE1$*$E1$_b$, KR1$*$K1$_b$, KR2$*$K2$_b$ and KR3$*$K3$_b$, E1$_b$, K1$_b$, K2$_b$ and K3$_b$ being defined for a reference multimedia message with a defined retention probability, determining that the multimedia message retention probability is higher than reference multimedia message retention probability when BRec is higher than Rec, or lower than reference multimedia message retention probability when BRec is lower than Rec.

In one particular case, the reference multimedia message may be presented to several users, and the reference multimedia message retention probability may be calculated as amount of users who retained the message, divided by the total amount of users both having retained or not the reference multimedia message.

In one particular case, the reference multimedia message may be a promotional video. In one particular case, at BRec =2.102, the multimedia message memorability probability may be set as 50%.

In one particular case, the multimedia message may be presented to several users, and E1, K1, K2 and K3 may be the average values of E1, K1, K2 and K3 calculated for all users.

In one particular case, during the assessment of memorability there may be no exclusion of artifacts from electroencephalograms due to spontaneous user behavior. In one particular case, the artifact due spontaneous user behavior may be an eye blink artifact.

In one particular case, for additional multimedia message, subsequent to the results of presentation to at least 10 users, the average BRec and the BRec defined for user may be considered unreliable if the divergence of average BRec and BRec defined for the users is more than 20% with respect to average BRec.

In one particular case, the user's eyelids' position may be registered via the video monitoring devices, and the results at the closed position of the user's eyelids lasting for more than 2 seconds may be excluded from the electroencephalograms.

Probability calculation functional relation based on the measurement results may be derived based on the results of machine learning using for example the questionnaire as learning sequence result.

Additional parameter for machine learning during memorability calculation may include percentage growth of sales for goods that were advertised in promotional videos that were the subjects of research.

The usage of formula:

$$P = \frac{1}{\left(1 + e^{(2.102 - KE1*E1 - KR1*K1 - KR2*K2 - KR3*K3)}\right)}$$

showed higher integrity of memorability probability calculation results compared with machine learning results.

Also, during research it has been discovered that multimedia messages (videos) that were different by just one of the researched indexes (E1, K1, K2 or K3) have different memorability levels, and differences in video appreciation, shown in other electroencephalogram's indexes, have not exhibited dependencies with videos memorability.

Herewith, 10% difference in E1 corresponded to probability change from 55 to 45 percent, 10% difference in K1 - from 55 to 50%, 10% difference in K2 - from 55 to 53 %, and K3 - from 55 to 54%. For other probability ranges there were different modules of probability variations, but general trend of dependency persisted.

In one particular case the users (in particular, target group) are the group of people who buy/consume the specified (examined) product (good, service etc.), that represents the specified market's consumer segment or group of several segments. The target group, as a rule, has certain properties: social and age, geographic, psychological, consumer etc.

In one particular case the target group excludes the following users: respondents (and/or their family members) working in advertising/PR, marketing/marketing research, manufacturing or sales of the researched products, television, radio, press, sociology, psychology and/or medicine; respondents that endured cerebropathy, head injuries, convulsive attacks, epistaxis and/or respondents with ophtalmopathy.

**Fig. 4a** and **4b** show the sample variant of electrodes' supports.

The individual case of reading the electroencephalogram **120A** is the multi-electrode electroencephalograph. The tool for electroencephalogram's reading **120** (particularly, electroencephalograph) may be the integrated part of neuro-fitting which provides the fixing at user **105** head the electrodes **115** in positions described in earlier in the present description. In one particular case there performed, the electrodes support is adjusted (particularly, electrodes **115** position change) such that all electrodes are tightly fit one the user **105** head skin to improve the proper EEG signal acquisition.

**Fig. 5** shows a variant of the present system implementation, including the user equipment.

The user equipment shown on **Fig. 5** is associated with one user, in particular, to user **105**. The system includes the computer **130**, the tool for electroencephalogram's reading **120**, the electrodes **115** linked therewith, and the display **110**, though it should be understood that user equipment or system's local part may belong to at least other user. The number of users in the present description (as well as aforementioned computers, tools, modules, electrodes, displays etc.) is not limited in one particular case and may depend on: the speed of connection of the computer or any other part of the described system (for example in case when system includes - or is linked to - computing devices, tools, modules etc. allowing to perform connection with such devices, tools, modules etc.), with Internet network (system); the data processing rate of described devices, tools, modules etc. and their other specifications. For the one particular case of implementation there will be performed the detection of EEG response to presentation of multimedia message.

In one particular case the multimedia message generation module **513** performs playback (particularly, generation) of at least one multimedia message displayed on display **110** for user **105**. The parameters of multimedia messages and their presentation (such as for example the number of multimedia messages presented, start and end time of presentation etc.) are defined by multimedia message generation module **513**. In one particular case the entering of the multimedia message parameters mentioned earlier may be performed in the multimedia message generation module **513** by an operator, for example with multimedia message parameters setting module that may be part of the computer **130**, submodule of the multimedia message generation module **513** or may be linked with them in any known way. The multimedia message generation module **513** may also perform the data transmission to data processing and analysis module **523** for later processing. The data transmitted may be the specifications (parameters) of the multimedia messages generated (and presented), which may be used by data processing and analysis module **523** as described in the present description.

In one particular case the tool for electroencephalogram reading (registration) **120** using connected electrodes **115** performs the reading of electroencephalogram (EEG), particularly, for registration of user's **105** brain activity to presented multimedia messages. The registered electroencephalogram transmits to data processing and analysis module **523** of computer **130** via wired and/or wireless data transmitting devices. In one particular case before transmission to data processing and analysis module **523** the registered electroencephalogram may be preprocessed by the tool for electroencephalogram's reading **120** or, at least, by one of its parts - for example, by preprocessing module. The preprocessing mentioned may include, for example, the decomposition of one signal registered by the tool for electroencephalogram's reading **120** to several signals, extraction of one signal from the group of signals, averaging (period), noise removal at least for one signal etc. In one particular case such pre-processing may be performed either by computer **130** (for example, by data processing and analysis module **523**), and/or by other module (for example, by data preprocessing module that is a part of the computer **130**).

The data transmitted to data processing and analysis module **523** (in particular, transmitted by the tool for electroencephalogram's reading **120**) may be analyzed (and/or processed) by such module.

In one particular case the data processing and analysis module **523** may perform the multimedia message memorability calculation as described in the present description.

The transmitted to the computer **130** data, also the data from data processing and analysis module **523**, may be stored in computer's **130** database at a data storage device (particularly, at data storage). The database may also store the configuration parameters described in the present description, for example related to users, multimedia messages, etc. The aforementioned database may be hierarchical, object, object-oriented, document-oriented, object-relational, relational, network and/or functional database, either of which may centralized, localized, distributed, heterogeneous, homogenous, fragmented (partitioned), replicated, spatial, temporal, spatial-temporal, cyclic, very large database etc., wherein for database management, creation and usage there may be used different DataBase Management Systems (DBMS). In one particular case the aforementioned storage may be a temporary storage memory (for example, random access memory RAM), a persistent repository (for example, (programmable) read-only memory ROM), among other, realized at least as one microchip or as microchip set etc. Also, the data may be stored in a repository, at least as one file (particularly, in text file), or the data may be stored in any known present data (information) storage format or in format that may arise in future.

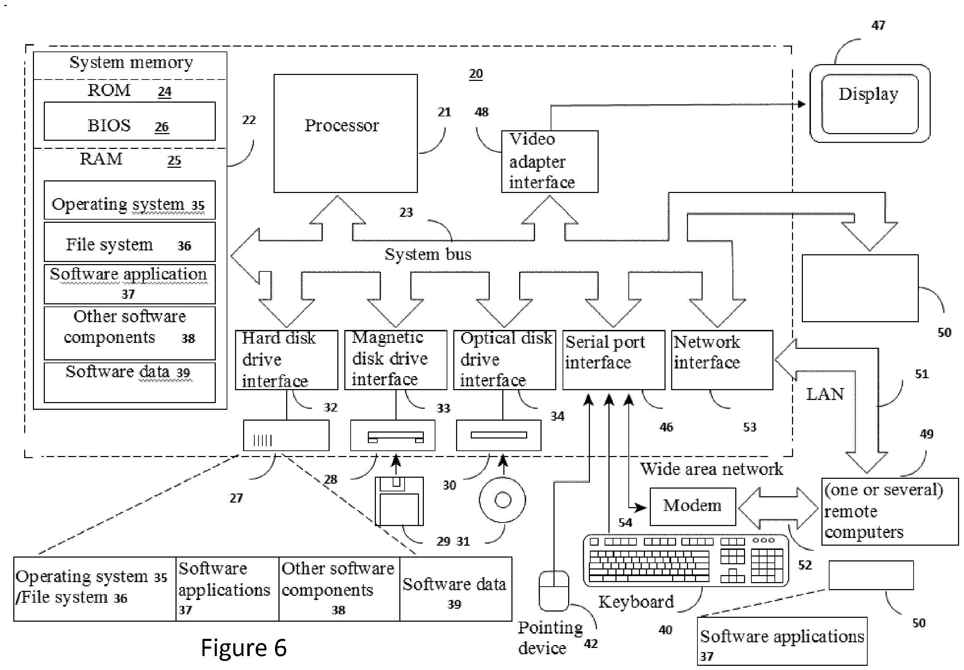

**Fig. 6** shows an example of computer system of general purpose, which includes multifunctional computing device in the form of computer **20** or server, or mobile (computing) device, or the module of the system described in the present description, that in one particular case may be a terminal (computing) devices (for example, user or operator terminal, etc.), comprising a processor **21**, a system memory **22** and a system bus **23**, that performs connection of different components of system, including the system memory with processor **21**.

The system bus **23** may be of any type of different bus structures, including memory bus or memory controller, peripheral bus and local bus, with any bus architecture. The system memory **22** includes read-only memory ROM **24** and random access memory RAM **25**. At ROM **24** there stored basic input-output system BIOS **26**, comprising base subprograms which render assistance to exchange of data between elements of computer **20** - for example, at system's start.

The computer **20** also may include the hard disk drive **27** for reading and writing of hard drive (not shown), magnetic disk drive **28** for reading and writing of removable magnetic disks **29**, and optical disk drive **30** for reading and writing of optical disks **31** such as compact disk, digital video disk and other optical tools. Hard disk drive **27**, magnetic disk drive **28** and optical disk drive **30** are connected with system bus **23** via, correspondingly, hard disk drive interface **32**, magnetic disk drive interface **33** and optical disk drive interface **34**. The drives and their corresponding readable by the computer tools are providing the nonvolatile storage of readable by the computer instructions, data structures, software modules and other computer **20** data.

Although the sample configuration described herein uses a hard drive, removable magnetic disk **29** and removable optical disk **31**, the skilled reader will note that in usual operatic environment there may be also used other computer-readable means of data storage, that may be available via computer such as magnetic tapes, flash-memory devices, digital video discs, Bernoulli cartridges, random access memory RAM devices, read-only access memory ROM devices and similar.

Different software components, including the operating system **35**, may be stored at hard drive, magnetic disc **29**, optical disk **31**, RAM **24** or ROM **25**. The computer **20** includes the file system **36**, connected with operating system **35** or included in it, one or more software applications **37**, other software components **38** and software data **39**. User may enter the commands and information to computer **20** via input devices such as keyboard **40** and pointing device **42**. Other input devices (not shown) may include microphone, joystick, gamepad, satellite antenna, scanner or any other device.

Aforementioned (and other) input devices are connected with the processor **21** usually via serial port interface **46** connected with system bus, but they may be connected via different interfaces, such as parallel port, game port or universal serial bus (USB). Display **47** or other type of visual display device also connected with system bus **23** via, for example video adapter interface **48**. In addition to display **47**, the personal computers usually includes other peripheral output devices (not shown), such as printers.

The computer **20** may work in networked environment via logical connections with one or more remote computers **49**. A remote computer (or computers) **49** may be a different computer, server, router, network PC, peer-to-peer device or other node of the single network, and usually includes the majority or all elements described above, in relation to computer **20**, though only the information storage device **50** of the remote computer **49** is shown. Logical connections include local area network LAN **51** and wide area network WAN **52**. Such network environments are the usual thing for enterprises, intranet, Internet.

Computer **20** uses a LAN network to connect with local network **51** via network interface or adapter **53**. Computer **20** used in WAN network usually use modem **54** or other devices to connect with wide area network **52** such as Internet.

The modem **54** that may be internal or external device connected with system bus **23** via serial port interface **46**. In network environment the software components and its parts described in relation to computer **20** may be stored at remote data storage device. It is necessary to take into consideration that all shown network connections are typical and there exists other communication methods for computers.

In summary, the information presented in this Description is just an example and the scope of present technology, described by its formula, is not limited to it. The expert skilled in the art will clearly see that there are other implementation variants for this present technology, complying with the present description's gist and scope.

**Claims**

**1.** The measurement method of memorability for multimedia message, that is:

via "10-20 system" use and reference electrodes installed at user's earlobes or mastoids, there must be installed electrodes at the points C3, C4, T3, T4, P3 and F4 (identified according to 10-20% international electrodes arrangement scheme) on user's head;

user's brain activity index will be registered during multimedia message presentation to the user, based on registration results, there will be determined the values for:

leads entropy (E1) for C3;

leads' first coherence (K1), that is equal to leads' coherence for T3 and T4 electrodes within the range 30-45 Hz;

leads' second coherence (K2), that is equal to leads' coherence for C4 and T4 electrodes within the range 4-8 Hz;

leads' third coherence (K3) that is equal to leads' coherence for P3 and F4 electrodes within the range 8-13 Hz;

there will be specified the memorability's weighting factor (KE1) for the leads entropy;

there will be specified first, second and third memorability's weighting factors (KR1, KR2 and KR3, respectively) for the first, second and third leads' entropy,

there will be defined the multimedia message's memorability (Rec) via calculation of the sum of productions $KE1*E1$, $KR1*K1$, $KR2*K2$ and $KR3*K3$ for E1, K1, K2 and K3, calculated for the multimedia message.

2. Method of claim 1, **characterized in that**: there will be estimated the base memorability (BRec) via calculation of the sum of productions $KE1*E1$, $KR1*K1$, $KR2*K2$ and $KR3*K3$ for E1, K1, K2 and K3, calculated for the reference multimedia message with predefined retention probability, accepting that multimedia message retention probability is higher than reference multimedia message retention probability at BRec higher than Rec, and lower than reference multimedia message retention probability at BRec lower than Rec.

3. Method of claim 2, **characterized in that**: the reference multimedia message will be presented to several users, and the reference multimedia message retention probability will be calculated as amount of users who retained the message, divided by the total amount of users both retained and not retained the reference multimedia message.

4. Method of claim 1, **characterized in that**: the multimedia message is the promotional video.

5. Method of claim 1, **characterized in that**: there will be chosen KR1<KR3<KR2<KE1.

6. Method of claim 5, **characterized in that**: there will be chosen KR1 = $0,5\pm30\%$, KR3= $1\pm30\%$, KR2 = $2\pm30\%$, KE1 = $4\pm30\%$.

7. Method of claim 2, **characterized in that**: the multimedia message retention probability will be 50% at BRec=2.102.

8. Method of claim 7, **characterized in that**: the multimedia message retention probability (P) will be determined from such formula:
$P=1/(1+\exp(2.102-KE1*E1-KR1*K1-KR2*K2-KR3*K3)$, where *exp* is power function.

9. Method of claim 1, **characterized in that**: the multimedia message will be presented to the user in the sequence of messages that includes additional multimedia messages; herein the additional multimedia messages will be placed at the start and at the end of sequence.

10. Method of claim 9, **characterized in that**: the sequence of messages includes pauses between additional multimedia messages, also between the additional multimedia messages and multimedia message.

11. Method of claim 10, **characterized in that**: the pauses will be filled by neutral videoclips.

12. Method of claim 1, **characterized in that**: during the assessment of memorability there will be no exclusion of artifacts from electroencephalograms due user's spontaneous behavior.

13. Method of claim 1, **characterized in that**: there will be registered the user's eyelids' position via the video monitoring devices, and the results at the closed position of the user's eyelids lasting for more than 2 seconds will be excluded from the electroencephalograms.

14. Method of claim 9 and claim 2, **characterized in that**: for the additional multimedia message, subsequent to the results of presentation to at least 10 users, there defined the average BRec and BRec defined for user, shall consider the unreliable if the divergence of average BRec and BRec defined for user is more than 20% with respect to average BRec.

Figure 1

Figure 2

314 — The arrangement of the electrodes

324 — The brain activity indexes will be registered during multimedia messages' presentation to the user

334 — Determination of the value for leads entropy (EI) for C3 electrode

344 — Determination of the leads' first coherence (K1)

354 — Determination of the leads' second coherence (K2)

364 — Determination of the leads' third coherence (K3)

374 — Specification of the recall's weighting factor for the entropy (KEI)

384 — Specification of the first, second and third recall's weighting factors, respectively, for K1, K2 and K3 (KR1, KR2 and KR3, respectively)

394 — The definition of the multimedia message's recall (Rec)

Figure 3

rear view

Figure 4a

side view

Figure 4b

Figure 5

Figure 6

## INTERNATIONAL SEARCH REPORT

| International application No. |
| --- |
| PCT/RU 2018/000848 |

**A.   CLASSIFICATION OF SUBJECT MATTER**
A61B 5/16 (2006.01); A61B 5/0408 (2006.01)

According to International Patent Classification (IPC) or to both national classification and IPC

**B.   FIELDS SEARCHED**

Minimum documentation searched (classification system followed by classification symbols)
A61B 5/16, 5/0408

Documentation searched other than minimum documentation to the extent that such documents are included in the fields searched

Electronic data base consulted during the international search (name of data base and, where practicable, search terms used)
PatSearch (RUPTO Internal), USPTO, PAJ, Espacenet, Information Retrieval System of FIPS

**C.   DOCUMENTS CONSIDERED TO BE RELEVANT**

| Category* | Citation of document, with indication, where appropriate, of the relevant passages | Relevant to claim No. |
| --- | --- | --- |
| D, A | CN 103500184 B (NORTHWESTERN POLYTECHNIC AL UNIVERSITY) 24.05.2017 | 1-14 |
| D, A | CN 102855630 A (UNIV NORTHWESTERN POLYTECHNIC) 02.01.2013 | 1-14 |
| A | CN 103440496 B (NORTHWESTERN POLYTECHNIC AL UNIVERSITY) 13.07.2016 | 1-14 |
| A | US 2012/0130800 A1 (ANANTHA PRADEEP et al.) 24.05.2012 | 1-14 |

☐   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 25 July 2019 (25.07.2019) | 22 August 2019 (22.08.2019) |

| Name and mailing address of the ISA/ RU | Authorized officer |
| --- | --- |
| | |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2018000848 W
- RU 2018145585
- CN 103500184
- CN 102855630